# EUROPEAN PATENT APPLICATION

(11) **EP 2 131 437 A1**
(43) Date of publication of application: **09.12.2009**
(21) Application number: 08721091.0
(22) Date of filing: 29.02.2008
(51) Int. Cl.: H01M 8/16, C12N 11/02, G01N 27/327, H01M 4/90, H01M 8/00

(54) **ENZYME IMMOBILIZED ELECTRODE, FUEL CELL, ELECTRONIC EQUIPMENT, ENZYME REACTION UTILIZATION APPARATUS, AND ENZYME IMMOBILIZED BASE**

(30) Priority: 23.03.2007 JP 2007077753
(71) Applicant: Sony Corporation, Minato-ku Tokyo 108-0075 (JP)
(72) Inventor: KAKUTA, Masaya, Tokyo 108-0075 (JP); SAKAI, Hideki, Tokyo 108-0075 (JP); NAKAGAWA, Takaaki, Tokyo 108-0075 (JP); TOKITA, Yuichi, Tokyo 108-0075 (JP)
(74) Representative: Müller - Hoffmann & Partner
(86) International application number: PCT/JP2008/053671
(87) International publication number: WO 2008/117630

(57) **Abstract**

An enzyme-immobilized electrode includes an electrode (11) composed of porous carbon or the like, a phospholipid layer (12) on the electrode (11), and enzymes (13, 14) immobilized onto the phospholipid layer (12). The enzymes (13, 14) are, for example, diaphorase and glucose dehydrogenase. An intermediate layer composed of a protein or the like may be provided between the electrode (11) and the phospholipid layer (12). By using the enzyme-immobilized electrode as a negative electrode or a positive electrode in a fuel cell using an enzyme, one or a plurality of types of enzymes can be immobilized at optimal positions on the electrode, and thus, there are provided a highly efficient enzyme-immobilized electrode and a highly efficient fuel cell using the enzyme-immobilized electrode.

## Description

### Technical Field

The present invention relates to an enzyme-immobilized electrode, a fuel cell, an electronic device, an apparatus utilizing enzyme reaction, and an enzyme-immobilized substrate, and is suitable for application, for example, to a biofuel cell using an enzyme, and various devices, apparatuses, systems, etc. using such a biofuel cell as the power source.

### Background Art

Fuel cells have a structure in which a positive electrode (oxidizer electrode) and a negative electrode (fuel electrode) are opposed to each other with an electrolyte (proton conductor) therebetween. In conventional fuel cells, the fuel (hydrogen) supplied to the negative electrode is oxidized and separated into electrons and protons (H⁺); the electrons are delivered to the negative electrode; and H⁺ moves through the electrolyte to the positive electrode. At the positive electrode, the H⁺ reacts with oxygen supplied from the outside and electrons transmitted from the negative electrode through an external circuit to generate H₂O.

As described above, fuel cells are highly efficient power-generating devices which convert the chemical energy possessed by a fuel directly into electrical energy, and are capable of extracting the chemical energy possessed by fossil energy, such as natural gas, oil, or coal, as electrical energy, regardless of the place of use or time of use, with high conversion efficiency. Therefore, conventionally, research and development has been actively carried out on fuel cells for the application in large-scale power generation, etc. For example, it has been actually proved that fuel cells can be installed in space shuttles and are capable of supplying electrical power as well as water for the crew and that fuel cells are clean power-generating devices.

Furthermore, in recent years, fuel cells, such as solid polymer fuel cells, which have a relatively low operating temperature range from room temperature to about 90°C, have been developed and have been receiving attention. Therefore, not only applications in large-scale power generation, but also applications in small systems, such as power sources for running automobiles and portable power sources for personal computers and mobile devices, have been sought after.
As described above, fuel cells are believed to have a wide range of applications from large-scale power generation to small-scale power generation, and have been receiving much attention as highly efficient power-generating devices. However, in fuel cells, usually, natural gas, oil, coal, or the like is converted into hydrogen gas by a reformer, the hydrogen gas being used as a fuel, which gives rise to various problems, such as consumption of limited resources, need to heat at high temperatures, and need of a catalyst composed of an expensive noble metal, such as platinum (Pt). Furthermore, even in the case where hydrogen gas or methanol is directly used as a fuel, handling thereof requires care.

Under these circumstances, focusing on the fact that biological metabolism taking place in living beings is a highly efficient energy conversion mechanism, its application to a fuel cell has been proposed. Here, biological metabolism includes respiration, photosynthesis, etc. taking place in microorganism cells. Biological metabolism has a characteristic in that its power generation efficiency is very high, and reaction proceeds under mild conditions at about room temperature.
For example, respiration is a mechanism in which nutrients, such as saccharides, fats, and proteins, are taken into microorganisms or cells, the chemical energy thereof is converted into oxidation-reduction energy, i.e., electrical energy, by reducing nicotinamide adenine dinucleotide (NAD⁺) to nicotinamide adenine dinucleotide reduced (NADH) in the process of generating carbon dioxide (CO₂) through a glycolytic pathway and a citric acid (TCA) cycle including many enzyme reaction steps, and, furthermore, in an electron transport system, the electrical energy of the NADH is directly converted into the electrical energy of a proton gradient, and also, oxygen is reduced to generate water. The electrical energy obtained here generates, through an adenosine triphosphate (ATP) synthase, ATP from adenosine diphosphate (ADP), and the ATP is used for reactions required for the growth of microorganisms and cells. Such energy conversion takes place in cytosol and mitochondria.

Furthermore, photosynthesis is a mechanism in which, in the process of taking in light energy, and converting light energy into electrical energy by reducing nicotinamide adenine dinucleotide phosphate (NADP⁺) to nicotinamide adenine dinucleotide phosphate reduced (NADPH) through an electron transport system, water is oxidized to generate oxygen. The electrical energy is used for a carbon immobilization reaction
in which CO₂ is taken in and for synthesis of carbohydrates.
As a technology in which biological metabolism as described above is used for a fuel cell, a microbial cell has been reported, in which electrical energy generated in microorganisms is taken out of the microorganisms through an electron mediator, and the electrons are delivered to an electrode to obtain an electric current (for example, refer to Japanese Unexamined Patent Application Publication No. 2000-133297).

However, in microorganisms and cells, many unnecessary functions are present in addition to the intended reaction, i.e., conversion from chemical energy into electrical energy. Therefore, in the above-described method, chemical energy is consumed in undesired reactions, and sufficient energy conversion efficiency is not obtained.
Under these circumstances, fuel cells (biofuel cells) in which only a desired reaction is carried out using an enzyme have been proposed (for example, refer to Japanese Unexamined Patent Application Publication No. 2003-282124, Japanese Unexamined Patent Application Publication No. 2004-71559, Japanese Unexamined Patent Application Publication No. 2005-13210, Japanese Unexamined Patent Application Publication No. 2005-310613, Japanese Unexamined Patent Application Publication No. 2006-24555, Japanese Unexamined Patent Application Publication No. 2006-49215, Japanese Unexamined Patent Application Publication No. 2006-93090, Japanese Unexamined Patent Application Publication No. 2006-127957, Japanese Unexamined Patent Application Publication No. 2006-156354, and Japanese Unexamined Patent Application Publication No. 2007-12281). In such biofuel cells, a fuel is decomposed by an enzyme and separated into protons and electrons. Biofuel cells using, as fuels, alcohols, such as methanol and ethanol; monosaccharides, such as glucose; or polysaccharides, such as starch, have been developed.

In such fuel cells, it is known that immobilization/arrangement of the enzyme with respect to the electrode is very important. Furthermore, it is also known that there is a need for effective presence of an electron mediator, which has a function of transporting electrons, together with the enzyme. As a conventional enzyme immobilization method, a polyion complex technique has been mainly used and developed, in which a positively charged polymer, a negatively charged polymer, and an enzyme are mixed at an appropriate ratio and applied onto an electrode composed of porous carbon or the like, and thereby, while maintaining adhesion with the electrode, the immobilized film is stabilized.

### Disclosure of Invention

However, the above-described immobilization method using the polyion complex largely depends on the physicochemical properties, in particular, the charge, of the enzyme, and there is fear that changes may occur constantly due to changes in the external solution, environmental changes during measurement, etc. Furthermore, in general, enzymes have low resistance to heat. In the process of altering an enzyme toward practical use of a biofuel cell, the physicochemical properties of the enzyme itself may change, and in each case, it is necessary to optimize the immobilized film formation method, which is troublesome. Furthermore, when it is desired to extract more electrons from the fuel, more enzymes are required. In the case where these enzymes are immobilized, a great deal of labor is required to optimize the conditions for immobilization. When attention is focused on the electron transport system in vivo, it is known that all the proteins that participate in the electron transport system are not necessarily water-soluble proteins, and membrane proteins and membrane-bound proteins are also contained therein. It is important that proteins be present in the right place when electrons are extracted at high efficiency.

Meanwhile, when a biomolecule is regarded as an electronic device, bioaffinity to an electrode composed of a silicon-based material, metal, carbon, or the like, which is used as its substrate, becomes a problem. For example, proteins as a kind of biomacromolecule often have a higher-order structure in order to efficiently fulfill their functions, and are known be denatured on the surface of a solid, such as the substrate of an electronic device, and at the interface between a liquid and a gas. Since biomacromolecules are handled differently from one another, solving the above problem is a key to improving device characteristics. Furthermore, when a plurality of enzymes are used in a biomimetic-type electronic device, it is very difficult to arrange the individual enzymes at optimal positions.

Accordingly, the problem to be solved by the present invention is to provide a highly efficient enzyme-immobilized electrode in which one or a plurality of types of enzymes can be immobilized at optimal positions on the electrode, a highly efficient fuel cell using the enzyme-immobilized electrode, an electronic device using the fuel cell, a highly efficient apparatus utilizing electrode reaction using the enzyme-immobilized electrode, and an enzyme-immobilized substrate including an enzyme-immobilized electrode, etc.

The present inventors have conducted diligent studies in order to solve the problem described above and, as a result, have found that a structure in which enzymes are immobilized onto a phospholipid layer disposed on an electrode or a structure in which enzymes are immobilized onto a phospholipid layer or a polyion complex disposed on an electrode through an intermediate layer composed of a protein or the like is effective. The effectiveness has been verified by experiments, and thus, the present invention has been devised.

That is, in order to solve the problem described above, a first invention is an enzyme-immobilized electrode characterized by including an electrode, a phospholipid layer on the electrode, and an enzyme immobilized onto the phospholipid layer.

As the material for the electrode, various types of materials can be used. For example, carbon-based materials, such as porous carbon, carbon pellets, carbon felt, and carbon paper, are used. As the material for the electrode, a porous conductive material including a skeleton composed of a porous material and a material containing as a main component a carbon-based material and covering at least a portion of the surface of the skeleton can also be used. The porous conductive material can be obtained by coating at least a portion of the surface of a skeleton, which is composed of a porous material, with a material which contains a carbon-based material as a main component. The porous material constituting the skeleton of the porous conductive material may be basically any material as long as the skeleton can be stably maintained even if the porosity is high, and regardless of presence or absence of conductivity. As the porous material, preferably, a material having high porosity and high conductivity is used. Specific examples of such a porous material having high porosity and high conductivity that can be used include metal materials (metals or alloys) and carbon-based materials with a strengthened skeleton (improved brittleness). When a metal material is used as the porous material, various choices are possible because the state stability of the metal material varies depending on the use environment, such as the pH and electric potential of a solution. For example, a metal foam or alloy foam composed of nickel, copper, silver, gold, a nickel-chromium alloy, stainless steel, or the like, is one of easily available materials. Besides the metal materials and carbon-based materials described above, resin materials (e.g., sponge-like materials) can also be used as the porous material. The porosity and pore diameter (minimum pore diameter) of the porous material are determined according to the porosity and pore diameter required for the porous conductive material in consideration of the thickness of the material containing as a main component a carbon-based material used for coating of the surface of the skeleton composed of the porous material. The pore diameter of the porous material is generally 10 nm to 1 mm, and typically 10 nm to 600 µm. In the meantime, as the material used for coating of the surface of the skeleton, it is necessary to use a material that has conductivity and that is stable at an estimated working potential. Here, as such a material, a material containing a carbon-based material as a main component is used. The carbon-based material generally has a wide potential window and, in many cases, has chemical stability. Specific examples of the material containing a carbon-based material as a main component include materials composed of only a carbon-based material and materials containing a carbon-based material as a main component and a small amount of a sub-material selected according to the characteristics required for the porous conductive material, etc. Examples of the latter materials include a material including a carbon-based material to which a highly conductive material, such as a metal, is added in order to improve electric conductivity, and a material to which a function other than conductivity is provided, for example, a material including a carbon-based material to which a polytetrafluoroethylene-based material or the like is added in order to impart surface water repellency. There are various types of carbon-based materials, and any carbon-based material may be used. The carbon-based material may be elemental carbon or may be a material in which another element is added to carbon. In particular, the carbon-based material is preferably a fine powder carbon material having high conductivity/high surface area. Specific examples of the carbon-based material that can be used include materials imparted with high conductivity, such as KB (Ketjenblack), and functional carbon materials, such as carbon nanotubes and fullerenes. As a method for coating with the material containing the carbon-based material as a main component, any coating method may be used as long as the surface of the skeleton composed of the porous material can be coated using an appropriate binder as necessary. The pore diameter of the porous conductive material is selected at such a size that a solution containing a substrate, etc. easily passes through the pores, and is generally 9 nm to 1 mm, more generally 1 µm to 1 mm, and still more generally 1 to 600 µm. In a state where at least a portion of the surface of the skeleton composed of the porous material is covered with the material containing the carbon-based material as a main component, or in a state where at least a portion of the surface of the skeleton composed of the porous material is coated with the material containing the carbon-based material as a main component, desirably, all pores communicate with one another or clogging is not caused by the material containing the carbon-based material as a main component.

The phospholipid layer is typically a lamellar phospholipid bilayer, or a phospholipid aggregate, such as a liposome which is a spherical phospholipid bilayer, but the form thereof is not particularly limited. Examples of the phospholipid layer also include a structure in which multiple (two or more) phospholipid bilayers are stacked, and a multilayer liposome having a nested structure in which a small liposome is incorporated into a large liposome. In the structure in which multiple phospholipid bilayers are stacked and the multilayer liposome, it is possible to extract energy with higher density, and it is possible to obtain a highly efficient enzyme-immobilized electrode by using two or more phospholipid bilayers having different characteristics. Liposomes can be formed in various sizes ranging from a diameter of about 100 nm to a large diameter of 10 µm. As the phospholipid, basically, any phospholipid may be used, and either a glycerophospholipid or a sphingophospholipid may be used. Examples of the glycerophospholipid include, but are not limited to, phosphatidic acid, phosphatidylcholine (lecithin), phosphatidylethanolamine, phosphatidylserine, phosphatidylglycerol, and diphosphatidylglycerol (cardiolipin). Examples of the sphingophospholipid include, but are not limited to, sphingomyelin. A typical example of the phosphatidylcholine is dimyristoyl phosphatidylcholine (DMPC).
Preferably, in addition to the enzyme, an electron mediator is also immobilized onto the phospholipid layer, or a coenzyme is further immobilized. The enzyme is, for example, a decomposing enzyme and is, in particular, a decomposing enzyme that decomposes fuel. As necessary, another one or a plurality of types of enzymes may be contained in addition.

The enzyme-immobilized electrode may include an intermediate layer between the electrode and the phospholipid layer in order to stabilize immobilization of the phospholipid layer with respect to the electrode. As the intermediate layer, not only a biomacromolecule, such as a protein or DNA, but also a polyelectrolyte having both hydrophilic and hydrophobic properties, a material that can form a structure, such as a micelle, an inverse micelle, or a lamella, and a compound with a nanometer structure having one or a plurality of properties and high bioaffinity can be used. Examples of the protein that can be used include acidic proteins, such as, albumin, as a representative, alcohol dehydrogenase, lactate dehydrogenase, ovalbumin, and myokinase, and, in addition, lysozyme, cytochrome c, myoglobin, trypsinogen, and the like having an isoelectric point on the alkaline side. By allowing am intermediate layer composed of such a protein or the like to physically adsorb on the surface of the electrode, by immobilizing a phospholipid layer, such as a phospholipid bilayer or a liposome, or a polyion complex onto the intermediate layer, and by immobilizing an enzyme thereonto, it is possible to stably immobilize the enzyme with respect to the electrode, thus enhancing freedom in the combination of the material for the electrode and the material for immobilization.

A second invention is an enzyme-immobilized electrode characterized by including an electrode, an intermediate layer on the electrode, a phospholipid layer and/or a polyion complex on the intermediate layer, and an enzyme immobilized onto the phospholipid layer and/or the polyion complex.

As the polyion complex, various types can be used. For example, polyion complexes formed using polycations, such as poly-L-lysine (PLL), or salts thereof and polyanions, such as polyacrylic acid (e.g., sodium polyacrylate (PAAcNa)), or salts thereof can be used. An enzyme, a coenzyme, an electron mediator, etc. can be contained in the polyion complex.
In the second invention, except for those described above, what has been described in relation to the first invention is valid, as long as not against the nature thereof.

A third invention is a fuel cell having a structure in which a positive electrode and a negative electrode are opposed to each other with a proton conductor therebetween, an enzyme being immobilized onto the positive electrode and/or the negative electrode, the fuel cell being **characterized in that** the positive electrode and/or the negative electrode is an enzyme-immobilized electrode including an electrode, a phospholipid layer on the electrode, and an enzyme immobilized onto the phospholipid layer.

A fourth invention is a fuel cell having a structure in which a positive electrode and a negative electrode are opposed to each other with a proton conductor therebetween, an enzyme being immobilized onto the positive electrode and/or the negative electrode, the fuel cell being **characterized in that** the positive electrode and/or the negative electrode is an enzyme-immobilized electrode including an electrode, an intermediate layer on the electrode, a phospholipid layer and/or a polyion complex on the intermediate layer, and an enzyme immobilized onto the phospholipid layer and/or the polyion complex.

In the third and fourth inventions, as the fuel, various substances can be used and are selected according to need. Typical examples thereof include methanol, ethanol, monosaccharides, and polysaccharides. In the case where monosaccharides, polysaccharides, and the like are used as the fuel, typically, they are used in the form of a fuel solution in which they are dissolved in a conventionally known buffer solution, such as a phosphate buffer or a Tris buffer.
For example, in the case where a monosaccharide, such as glucose, is used as the fuel, preferably, an oxidase which promotes oxidation of the monosaccharide and decomposes it and a coenzyme oxidase which returns a coenzyme reduced by the oxidase to an oxidized form are immobilized, as enzymes, onto an enzyme-immobilized electrode used as the negative electrode. When the coenzyme is returned to the oxidized form by the action of the coenzyme oxidase, electrons are generated, and the electrons are transferred to the electrode from the coenzyme oxidase through the electron mediator. As the oxidase, for example, glucose dehydrogenase (GDH) is used. As the coenzyme, for example, nicotinamide adenine dinucleotide (NAD⁺) or nicotinamide adenine dinucleotide phosphate (NADP⁺) is used. As the coenzyme oxidase, for example, diaphorase (DI) is used. As the electron mediator, basically, any material may be used, and preferably, a compound having a quinone skeleton, in particular, a compound having a naphthoquinone skeleton is used. As the compound having a naphthoquinone skeleton, various naphthoquinone derivatives may be used. Specific usable examples include 2-amino-1,4-naphthoquinone (ANQ), 2-amino-3-methyl-1,4-naphthoquinone (AMNQ), 2-methyl-1,4-naphthoquinone (VK3), 2-amino-3-carboxy-1,4-naphthoquinone (ACNQ), and vitamin K1. As the compound having a quinone skeleton, besides the compound having a naphthoquinone skeleton, for example, anthraquinone or a derivative thereof may be used. As necessary, besides the compound having a quinone skeleton, one or two or more other compounds serving as an electron mediator may be contained in the electron mediator.

In the case where a polysaccharide (i.e., polysaccharide in a broad sense, referring to all carbohydrates which generate two or more molecules of monosaccharide through hydrolysis, and including oligosaccharides, such as disaccharides, trisaccharides, and tetrasaccharides) is used as the fuel, preferably, a decomposing enzyme which promotes decomposition, such as hydrolysis, of the polysaccharide and generates a monosaccharide, such as glucose is also immobilized, in addition to the above-described oxidase, coenzyme oxidase, coenzyme, and electron mediator. Specific examples of the polysaccharide include starch, amylose, amylopectin, glycogen, cellulose, maltose, sucrose, and lactose. These polysaccharides are composed of two or more monosaccharides bonded together, and each of these polysaccharides contains glucose as a monosaccharide serving as a bonding unit. In addition, amylose and amylopectin are components contained in starch, and starch is a mixture of amylose and amylopectin. In the case where glucoamylase is used as the decomposing enzyme for polysaccharides and where glucose dehydrogenase is used as the oxidase to decompose monosaccharides, a polysaccharide which may be decomposed to glucose with glucoamylase, for example, any one of starch, amylose, amylopectin, glycogen, and maltose, may be used as the fuel to generate electricity. In addition, glucoamylase is a decomposing enzyme which hydrolyzes α-glucan, such as starch, to produce glucose, and glucose dehydrogenase is an oxidase which oxidizes β-D-glucose to D-glucono-δ-lactone.

In the fuel cell in which cellulase is used as the decomposing enzyme and glucose dehydrogenase is used as the oxidase, cellulose which can be decomposed to glucose by cellulase can be used as the fuel. More particularly, cellulase is at least one of cellulase (EC 3.2.1.4), exo-cellobiohydrolase (EC 3.2.1.91), β-glucosidase (EC 3.2.1.21), and the like. In addition, glucoamylase and cellulase may be mixed for use as the decomposing enzyme. In such a case, since most of polysaccharides produced in the natural world can be decomposed, substances containing a large amount of these polysaccharides, for example, garbage, can be used as fuels.

Furthermore, in the fuel cell in which α-glucosidase is used as the decomposing enzyme and glucose dehydrogenase is used as the oxidase, maltose which is decomposed to glucose by α-glucosidase can be used as the fuel.
Furthermore, in the fuel cell in which sucrase is used as the decomposing enzyme and glucose dehydrogenase is used as the oxidase, sucrose which is decomposed to glucose and fructose by sucrase can be used as the fuel. More particularly, sucrase is at least one of α-glucosidase (EC 3.2.1.20), sucrose-αglucosidase (EC 3.2.1.48), β-fructofuranosidase (EC 3.2.1.26), and the like.
Furthermore, in the fuel cell in which β-galactosidase is used as the decomposing enzyme and glucose dehydrogenase is used as the oxidase, lactose which is decomposed to glucose and galactose by β-galactosidase can be used as the fuel.
As necessary, these polysaccharides serving as fuels may also be immobilized onto the negative electrode.

In particular, in the fuel cell in which starch is used as the fuel, a gel-like solidified fuel obtained by gelatinizing starch can also be used. In such a case, there can be employed a method in which gelatinized starch is brought into contact with the negative electrode having immobilized thereon an enzyme and others, or a method in which gelatinized starch is immobilized on the negative electrode, together with an enzyme and others. When such an electrode is used, the starch concentration on the negative electrode surface can be kept high compared with the case where starch dissolved in a solution is used. Therefore, the decomposition reaction by the enzyme is faster, and the output is improved. Furthermore, handling of the fuel is easier than the case of the solution, and the fuel supply system can be simplified. Moreover, it is not necessary to ban upside-down handling, and thus the fuel cell is very advantageous in use in mobile devices.

In the case where an enzyme is immobilized onto the positive electrode, the enzyme typically includes an oxygen-reducing enzyme. As the oxygen-reducing enzyme, for example, bilirubin oxidase, laccase, ascorbate oxidase, or the like can be used.
In such a case, preferably, in addition to the enzyme, an electron mediator is also immobilized onto the positive electrode. As the electron mediator, for example, potassium hexacyanoferrate, potassium octacyanotungstate, or the like is used. Preferably, the electron mediator is immobilized at a sufficiently high concentration, for example, at an average value of 0.64 × 10⁻⁶ mol/mm² or more.

In the case where an electrolyte containing a buffer substance (buffer solution) is used as a proton conductor, in order to provide a sufficient buffer capacity and fully exhibit the inherent ability of the enzyme during high-power operation, it is effective to set the concentration of the buffer substance contained in the electrolyte at 0.2 M to 2.5 M, preferably 0.2 M to 2 M, more preferably 0.4 M to 2 M, and still more preferably 0.8 M to 1.2 M. Any buffer substance may be used as long as it has a pKₐ of 6 to 9. Specific examples thereof include dihydrogen phosphate ions (H₂PO₄⁻), 2-amino-2-hydroxymethyl-1,3-propanediol (abbreviated as Tris), 2-(N-morpholino)ethanesulfonic acid (MES), cacodylic acid, carbonic acid (H₂CO₃), hydrogen citrate ions, N-(2-acetamido)iminodiacetic acid (ADA), piperazine-N,N'-bis(2-ethanesulfonic acid) (PIPES), N-(2-acetamido)-2-aminoethanesulfonic acid (ACES), 3-(N-morpholino)propanesulfonic acid (MOPS), N-2-hydroxyethylpiperazine-N'-2-ethanesulfonic acid (HEPES), N-2-hydroxyethylpiperazine-N'-3-propanesulfonic acid (HEPPS), N-[tris(hydroxymethyl)methyl]glycine (abbreviated as Tricine), glycylglycine, and N,N-bis(2-hydroxyethyl)glycine (abbreviated as Bicine). Examples of a material that generates dihydrogen phosphate ions (H₂PO₄⁻) include sodium dihydrogen phosphate (NaH₂PO₄) and potassium dihydrogen phosphate (KH₂PO₄). As the buffer substance, an imidazole ring-containing compound is also preferable. Specific examples of the imidazole ring-containing compound include imidazole, triazole, pyridine derivatives, bipyridine derivatives, and imidazole derivatives (histidine, 1-methylimidazole, 2-methylimidazole, 4-methylimidazole, 2-ethylimidazole, ethyl imidazole-2-carboxylate, imidazole-2-carboxaldehyde, imidazole-4-carboxylic acid, imidazole-4,5-dicarboxylic acid, imidazol-1-yl-acetic acid, 2-acetylbenzimidazole, 1-acetylimidazole, N-acetylimidazole, 2-aminobenzimidazole, N-(3-aminopropyl)imidazole, 5-amino-2-(trifluoromethyl)benzimidazole, 4-azabenzimidazole, 4-aza-2-mercaptobenzimidazole, benzimidazole, 1-benzylimidazole, and 1-butylimidazole). The pH of the electrolyte containing the buffer substance is preferably about 7, but generally may be any of 1 to 14.

The fuel cell can be used in all devices which require electric power and can have any sizes. For example, the fuel cell can be used in electronic devices, movable bodies (automobiles, two-wheeled vehicles, aircrafts, rockets, spacecrafts, etc.), power devices, construction machines, machine tools, power generation systems, and cogeneration systems, and the output, size, shape, type of the fuel, etc. are determined according to applications, etc.
In the third and fourth inventions, except for those described above, what has been described in relation to the first and second inventions is valid, as long as not against the nature thereof.

A fifth invention is an electronic device using one or a plurality of fuel cells, the electronic device being **characterized in that** at least one of the fuel cells has a structure in which a positive electrode and a negative electrode are opposed to each other with a proton conductor therebetween, an enzyme being immobilized onto the positive electrode and/or the negative electrode, and the positive electrode and/or the negative electrode is an enzyme-immobilized electrode including an electrode, a phospholipid layer on the electrode, and an enzyme immobilized onto the phospholipid layer.

A sixth invention is an electronic device using one or a plurality of fuel cells, the electronic device being **characterized in that** at least one of the fuel cells has a structure in which a positive electrode and a negative electrode are opposed to each other with a proton conductor therebetween, an enzyme being immobilized onto the positive electrode and/or the negative electrode, and the positive electrode and/or the negative electrode is an enzyme-immobilized electrode including an electrode, an intermediate layer on the electrode, a phospholipid layer and/or a polyion complex on the intermediate layer, and an enzyme immobilized onto the phospholipid layer and/or the polyion complex.

The electronic device according to each of the fifth and sixth inventions may be basically any electronic device, and includes both an electronic device of a portable type and an electronic device of a fixed type. Specific examples thereof include cellular phones, mobile devices (personal digital assistant (PDA) and the like), robots, personal computers (including both desktop type and notebook type), game machines, camcorder VTRs (video tape recorders), devices mounted on cars, household electric appliances, and industrial products.
In the fifth and sixth inventions, what has been described in relation to the first to fourth inventions is valid, as long as not against the nature thereof.

A seventh invention is an apparatus utilizing enzyme reaction including an enzyme-immobilized electrode, the apparatus utilizing enzyme reaction being **characterized in that** the enzyme-immobilized electrode includes an electrode, a phospholipid layer on the electrode, and an enzyme immobilized onto the phospholipid layer.

An eighth invention is an apparatus utilizing enzyme reaction including an enzyme-immobilized electrode, the apparatus utilizing enzyme reaction being **characterized in that** the enzyme-immobilized electrode includes an electrode, an intermediate layer on the electrode, a phospholipid layer and/or a polyion complex on the intermediate layer, and an enzyme immobilized onto the phospholipid layer and/or the polyion complex.

In each of the seventh and eighth inventions, the apparatus utilizing electrode reaction includes, in addition to the fuels cells described above, i.e., biofuel cells, biosensors (glucose sensors, etc.), bioreactors, and the like, and as the enzyme, enzymes are used according to individual purposes.
In the seventh and eighth inventions, except for those described above, what has been described in relation to the first to sixth inventions is valid, as long as not against the nature thereof.

A ninth invention is an enzyme-immobilized substrate characterized by including a substrate, a phospholipid layer on the substrate, and an enzyme immobilized onto the phospholipid layer.

A tenth invention is an enzyme-immobilized substrate characterized by including a substrate, an intermediate layer on the substrate, a phospholipid layer and/or a polyion complex on the intermediate layer, and an enzyme immobilized onto the phospholipid layer and/or the polyion complex.

In the ninth and tenth inventions, the substrate, which may have conductivity or non-conductivity, includes, besides electrodes, various types of substrates, such as a Si substrate and a metal substrate, and also includes electronic devices and the like.
With respect to proteins responsible for electron transport in vivo, there are three types: membrane proteins, membrane-bound proteins, and water-soluble proteins. The conventional enzyme immobilization method using the polyion complex technique has a drawback in that the three types of proteins having different physicochemical properties are immobilized in the same manner. Therefore, there is fear that functions/activities inherently possessed by proteins may be impaired. In contrast, according to the ninth and tenth inventions, an advantage can be obtained in that, by using the nature of proteins, it is possible easily localize membrane proteins into membranes, membrane-bound proteins to membrane surface layers, and water-soluble proteins to the outside or the inside. The electron transport system is not the only example, and among many reactions in vivo, such as photoreception, there are some reactions that use localization through phospholipid membranes in such a manner. Applications to such cases are assumed to be effective.
Furthermore, in addition to an enzyme-immobilized electrode using a phospholipid layer, by combining aggregates having one or two or more different properties, it is possible to produce pseudo-bodies having hierarchy. Thereby, a device having various functions can be realized. Furthermore, this can respond to stimuli and the like from the external world, and can also be used as an information processing device.
In the ninth and tenth inventions, except for those described above, what has been described in relation to the first to sixth inventions is valid, as long as not against the nature thereof.

In the present invention having the configurations described above, it is possible to immobilize one or a plurality of types of enzymes, stably and with high activity being maintained, at appropriate positions of phospholipid layers, such as phospholipid bilayers and liposomes.
Furthermore, it is possible to immobilize one or a plurality of types of enzymes, stably and with high activity being maintained, at appropriate positions of phospholipid layers or polyion complexes.

### Brief Description of Drawings

[Fig. 1] Fig. 1 is a schematic diagram showing an enzyme-immobilized electrode according to a first embodiment of the present invention.
[Fig. 2] Fig. 2 is a schematic diagram showing a liposome used in Example 1 of the present invention.
[Fig. 3] Fig. 3 is a confocal microscope image of a surface of an enzyme-immobilized electrode fabricated in Example 1 of the present invention.
[Fig. 4] Fig. 4 is a schematic diagram showing an enzyme-immobilized electrode according to a second embodiment of the present invention.
[Fig. 5] Fig. 5 is a schematic diagram showing an enzyme-immobilized electrode according to a third embodiment of the present invention.
[Fig. 6] Fig. 6 is a schematic diagram showing an enzyme-immobilized electrode according to a fourth embodiment of the present invention.
[Fig. 7] Fig. 7 is a schematic diagram showing a biofuel cell according to a fifth embodiment of the present invention.
[Fig. 8] Fig. 8 is a schematic diagram which schematically shows a detailed structure of a negative electrode of a biofuel cell according to the fifth embodiment of the present invention, an example of a group of enzymes immobilized onto the negative electrode, and electron transfer reaction by the group of enzymes.
[Fig. 9] Fig. 9 includes schematic diagrams showing a specific structural example of a biofuel cell according to the fifth embodiment of the present invention.

### Best Modes for Carrying Out the Invention

The embodiments of the present invention will be described below with reference to the drawings. In all the drawings relating to the embodiments, the same or corresponding parts are designated by the same reference numerals.

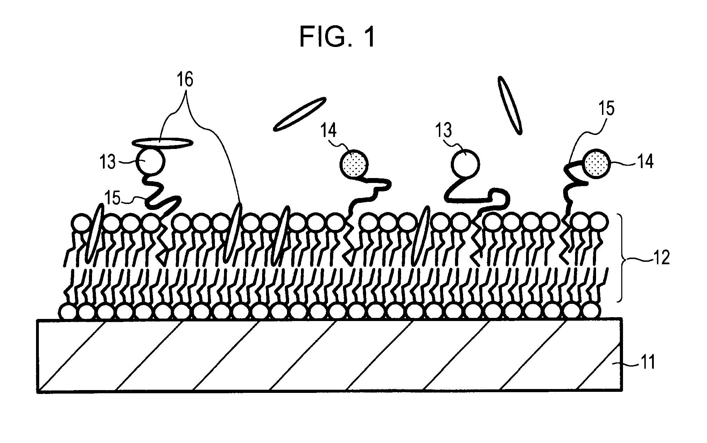

Fig. 1 shows an enzyme-immobilized electrode according to a first embodiment of the present invention.
As shown in Fig. 1, in the enzyme-immobilized electrode, a phospholipid bilayer 12 is immobilized by physical adsorption or the like onto an electrode 11 composed of porous carbon or the like. Two types of enzymes 13 and 14 are immobilized onto the phospholipid bilayer 12 through an anchor 15. Although the case where two types of enzymes 13 and 14 are immobilized is described here, the same applies to the case where three or more types of enzymes are immobilized. As the anchor 15, for example, a polyethylene glycol chain can be used, although not limited thereto. In addition to the enzymes 13 and 14, an electron mediator 16 is also immobilized onto the phospholipid bilayer 12.
The enzyme-immobilized electrode can be fabricated, for example, by a method in which a mixture of a phospholipid, the enzymes 13 and 14, and the electron mediator 16 is added dropwise or applied onto the electrode 11, followed by drying. <EXAMPLE 1>

Proteins are generally classified into three types: membrane proteins distributed in cell membranes, water-soluble proteins distributed in extracellular or intracellular solutions, and membrane-bound proteins lying in contact with cell membranes. Among them, membrane proteins and membrane-bound proteins are easily introduced into liposomes. Meanwhile, some proteins used for biofuels are often distributed in intracellular or extracellular solutions and have a relatively small amount of lipid-soluble sites.

Meanwhile, as the configuration of phospholipid aggregates, spherical liposomes in the form of phospholipid bilayers are well known. Most of membranes constituting living beings are composed of liposomes which are the phospholipid aggregates, and they are very good reaction fields for keeping enzymes, proteins, and, in particular, membrane proteins, to be stable and highly active. Accordingly, in Example 1, first, lipid-soluble sites are introduced into two types of enzymes 13 and 14, which are then introduced into liposomes that are thermodynamically stable phospholipid aggregates. Specifically, as the enzymes 13 and 14, diaphorase and glucose dehydrogenase, which are generally water-soluble, are used. Lipid-soluble sites are chemically introduced thereinto, and distributed, in an arbitrary quantity and at an arbitrary rate, in liposomes. A compound (manufactured by NOF Corporation, trade name: SUNBRIGHT OE-040CS) used in order to introduce lipid-soluble sites into diaphorase and glucose dehydrogenase includes sites for covalently binding to the diaphorase and glucose dehydrogenase, oleoyl groups which are sites to be introduced into phospholipid membranes, and polyethylene glycol chains that bind these two portions. The compound was prepared with reference to documents, etc.

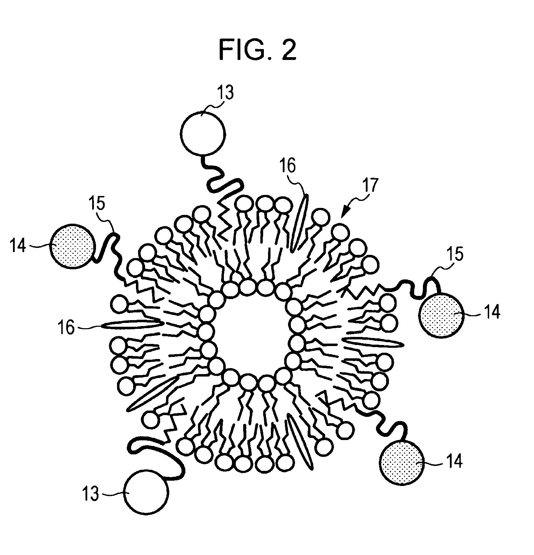

Liposomes can have different sizes and configurations depending on the formation method. Here, liposomes were formed by a sonication method. As the type of phospholipid, Egg yolk-derived phospholipid was used.
Proteoliposomes in which diaphorase and glucose dehydrogenase were arranged in liposomes were prepared by a method in which the liposomes prepared as described above, diaphorase and glucose dehydrogenase having chemically introduced lipid-soluble sites, and an electron mediator 16 were simultaneously mixed and then left to stand for several hours. In order to observe introduction of the diaphorase and glucose dehydrogenase into the liposomes, the diaphorase and glucose dehydrogenase were labeled with a fluorescent substance, and checked if water-soluble diaphorase and glucose dehydrogenase chemically provided with lipid-soluble sites were introduced into the liposomes. As a result, it was confirmed that both of them were introduced. Fig. 2 is a schematic diagram of the proteoliposome. Reference numeral 17 represents a liposome.

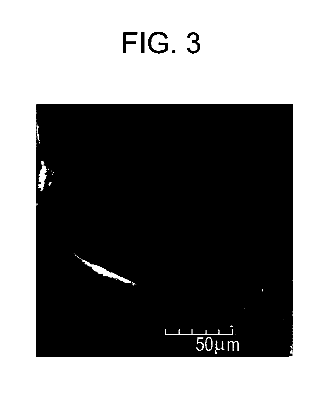

By adding the proteoliposomes prepared as described above dropwise onto an electronic device or a substrate, the liposomes can be immobilized. Although depending on the configuration of liposome, it is known that a phospholipid monolayer is formed on a water-repellent substrate, and a phospholipid bilayer is formed on a hydrophilic substrate. In Example 1, a porous carbon electrode which had been hydrophilized by ozonation treatment in advance was used as the substrate 11. Fig. 3 shows a confocal microscope image of a surface of an electrode when fluorescence-labeled diaphorase and glucose dehydrogenase were actually used. Fluorescence labeling was performed using a fluorescent substance that emits green light and a fluorescent substance that emits red light for diaphorase and glucose dehydrogenase, and as is evident from Fig. 3, both are believed to become adhered and fused to the surface of the porous carbon electrode.

The porous carbon electrode prepared as described above was thoroughly washed with a phosphate buffer to remove excess proteoliposomes, followed by drying. Thereby, an electrode for electrochemical measurement was obtained. Using the enzyme-immobilized electrode thus fabricated, chronoamperometry and cyclic voltammetry were performed in a glucose solution. In this case, an imidazole buffer solution was used as the buffer solution, and the glucose concentration was 400 mM. Cyclic voltammetry and constant potential measurement at 0.1 V were performed. As a comparative example, a conventional enzyme-immobilized electrode was used, in which enzymes were immobilized using polyion complexes formed using poly-L-lysine (PLL) and sodium polyacrylate (PAAcNa) (Comparative Example 1).
A catalytic current value was confirmed in each case. Results of constant potential measurement after one hour showed that the value was 2.99 mA/cm² in the enzyme-immobilized electrode of Example 1 and the value was 2.20 mA/cm² in the enzyme-immobilized electrode of Comparative Example 1. Thus, it was possible to confirm a high catalytic current value, with a significant difference.

According to the first embodiment, it is possible to obtain an enzyme-immobilized electrode in which a phospholipid bilayer 12 is immobilized onto an electrode 11, and two types of enzymes 13 and 14 and an electron mediator 16 are immobilized onto the phospholipid bilayer 12. In this enzyme-immobilized electrode, since the two types of enzymes 13 and 14 can be stably immobilized at optimal positions and at arbitrary ratio, efficiency is high, and a large catalytic current value can be obtained.

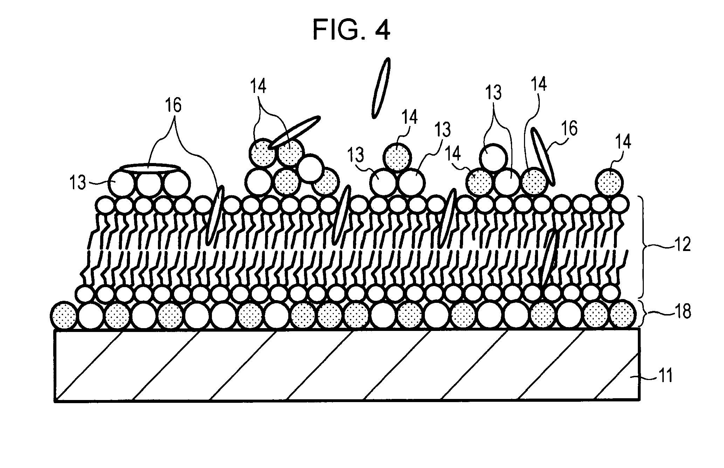

Fig. 4 shows an enzyme-immobilized electrode according to a second embodiment of the present invention.
As shown in Fig. 4, in the enzyme-immobilized electrode, an intermediate layer 18 composed of a protein or the like is immobilized by physical adsorption or the like onto an electrode 11 composed of porous carbon or the like, and a phospholipid bilayer 12 is immobilized by physical adsorption or the like onto the intermediate layer 18. Two types of enzymes 13 and 14 are immobilized onto the phospholipid bilayer 12. In addition to the enzymes 13 and 14, an electron mediator 16 is also immobilized onto the phospholipid bilayer 12.
The enzyme-immobilized electrode can be fabricated, for example, by a method in which the intermediate layer 18 is formed by immersing the electrode 11 in a solution containing a protein or the like, and a mixture of a phospholipid, the enzymes 13 and 14, and the electron mediator 16 is added dropwise or applied thereonto, followed by drying.

### <EXAMPLE 2>

As the electrode 11, a porous carbon electrode was used. The porous carbon electrode was activated by an ultraviolet (UV) ozonation treatment apparatus so that the surface became hydrophilic, then immersed in a phosphate buffer including about 500 µg/mL to 5 mg/mL of an enzyme, and left to stand overnight. Then, washing with a phosphate buffer and drying were performed. Thereby, a porous carbon electrode having the enzyme as an intermediate layer 18 was obtained.

Next, liposomes including ANQ as an electron mediator 16, diaphorase and glucose dehydrogenase as enzymes 13 and 14, and NADH as a coenzyme were added dropwise onto the porous carbon electrode having the enzyme as the intermediate layer 18, followed by drying. An enzyme-immobilized electrode was thereby fabricated. As controls, an enzyme-immobilized electrode was fabricated by activating a porous carbon electrode by an UV ozonation treatment apparatus to hydrophilize the surface, and then adding dropwise a liposome solution containing enzymes, etc. (Comparative Example 2), and an enzyme-immobilized electrode was fabricated, in which enzymes were immobilized using polyion complexes formed using PLL and PAAcNa (Comparative Example 1). Comparison was carried out electrochemically. In the measurement, using an imidazole buffer solution containing 400 mM of glucose, constant potential measurement at 0.1 V and cyclic voltammetry were performed. A distinct catalytic current value was obtained in each case. According to constant potential measurement after one hour, the value was 3.36 mA/cm² in the enzyme-immobilized electrode of Example 2, while the value was 2.29 mA/cm² in the enzyme-immobilized electrode of Comparative Example 1. Thus, a significant difference is recognized, and the excellent result is obtained. Furthermore, in the enzyme-immobilized electrode of Example 2, even when compared to Comparative Example 2 (1.96 mA/cm²), a significant difference is recognized, and the excellent result is obtained. Consequently, it is believed that, compared with the case where liposomes directly act on the electrode to form an enzyme reaction field, an enzyme-immobilized film can be stably formed in the case where an intermediate layer composed of a protein or the like is present. Furthermore, it is also believed that a phospholipid serves as a good enzyme reaction field. As is evident from the above, stabilization of an enzyme-immobilized film by liposomes can be promoted not only by performing hydrophilization treatment on the surface of the electrode by an UV ozonation treatment apparatus or the like, but also by introducing an intermediate layer of some kind of protein.
According to the second embodiment, the same advantages as those in the first embodiment can be obtained.

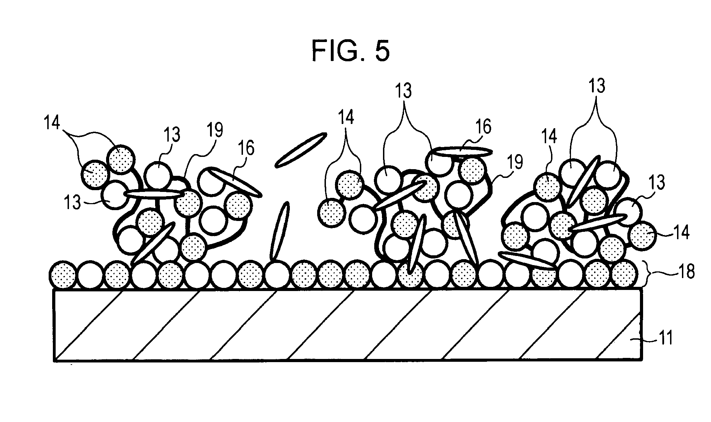

Fig. 5 shows an enzyme-immobilized electrode according to a third embodiment of the present invention.
As shown in Fig. 5, in the enzyme-immobilized electrode, an intermediate layer 18 composed of a protein or the like is immobilized by physical adsorption or the like onto an electrode 11 composed of porous carbon or the like, and polyion complexes 19 are immobilized onto the intermediate layer 18. Two types of enzymes 13 and 14 are immobilized onto the polyion complexes 19. In addition to the enzymes 13 and 14, an electron mediator 16 is also immobilized onto the polyion complexes 19.
The enzyme-immobilized electrode can be fabricated, for example, by a method in which the intermediate layer 18 is formed by immersing the electrode 11 in a solution containing a protein or the like, and a mixture of polycations, polyanions, the enzymes 13 and 14, and the electron mediator 16 is added dropwise or applied thereonto, followed by drying.

### <EXAMPLE 3>

As the electrode 11, a porous carbon electrode was used. The porous carbon electrode was activated by an ultraviolet (UV) ozonation treatment apparatus so that the surface became hydrophilic, then immersed in a phosphate buffer including about 500 µg/mL to 5 mg/mL of an enzyme and left to stand overnight. Then, washing with a phosphate buffer and drying were performed. Thereby, a porous carbon electrode having the enzyme as an intermediate layer 18 was obtained.

Next, a solution including ANQ as an electron mediator 16, diaphorase and glucose dehydrogenase as enzymes 13 and 14, NADH as a coenzyme, PLL as polycations, PLL as polyanions, and PAAcNa as polycations was added dropwise onto the porous carbon electrode having the enzyme as the intermediate layer 18, followed by drying. An enzyme-immobilized electrode was thereby fabricated. Using, as a control, an enzyme-immobilized electrode of Comparative Example 1, comparison was carried out electrochemically. In the measurement, using an imidazole buffer solution containing 400 mM of glucose, constant potential measurement at 0.1 V and cyclic voltammetry were performed. It was confirmed that a distinct catalytic current value can be obtained. Furthermore, the result of constant potential measurement at 0.1 V shows that the value of the enzyme-immobilized electrode of Example 3 after one hour was 3.12 mA/cm². Thus, a significant difference is recognized from Comparative Example 1 (2.29 mA/cm²), and the excellent result is obtained. Consequently, it is evident that, even in the case where an ion complex technique, which is a conventional technique, is used, a good electrochemical response to the electrode provided with the intermediate layer composed of a protein is shown.
Taking together with the results of Example 2, it is evident that stabilization of an enzyme-immobilized film by liposomes or the polyion complex technique can be promoted not only by performing hydrophilization treatment on the surface of the electrode by an UV ozonation treatment apparatus or the like, but also by introducing an intermediate layer of some kind of protein.
According to the third embodiment, the same advantages as those in the first embodiment can be obtained.

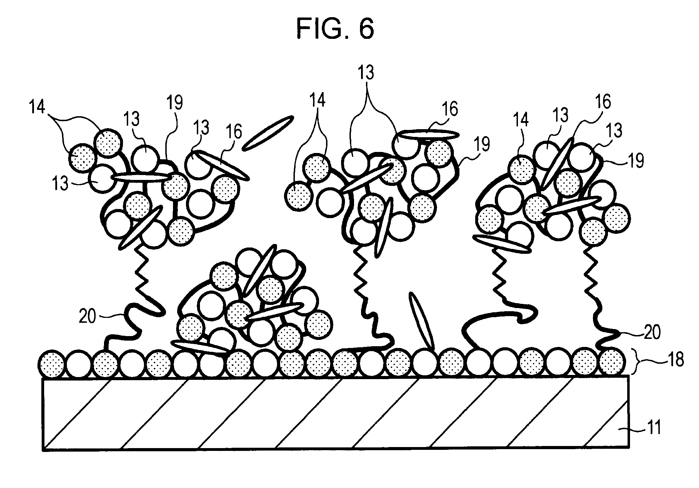

Fig. 6 shows an enzyme-immobilized electrode according to a fourth embodiment of the present invention.
As shown in Fig. 6, in the enzyme-immobilized electrode, an intermediate layer 18 composed of albumin is immobilized by physical adsorption or the like onto an electrode 11 composed of porous carbon or the like, and lipid-soluble functional group-containing molecules 20 are covalently bound to albumin of the intermediate layer 18. Polyion complexes having immobilized two types of enzymes 13 and 14 are immobilized with the molecules 20 being used as an anchor. In addition to the enzymes 13 and 14, an electron mediator 16 is also immobilized onto the polyion complexes 19.
The enzyme-immobilized electrode can be fabricated, for example, by a method in which the intermediate layer 18 is formed by immersing the electrode 11 in a solution containing albumin, the molecules 20 are covalently bound to the intermediate layer 18, and a mixture of polycations, polyanions, the enzymes 13 and 14, and the electron mediator 16 is added dropwise or applied thereonto, followed by drying.

### <EXAMPLE 4>

A porous carbon electrode was activated by a UV ozonation treatment apparatus so that the surface became hydrophilic, then immersed in an albumin solution (bovine-derived) (phosphate buffer), the concentration of which was adjusted to about 1%, and left to stand overnight. Then, the porous carbon electrode was washed with a phosphate buffer and dried. Thereby, an electrode 11 having albumin as the intermediate layer 18 was obtained. Furthermore, molecules 20 having fatty groups and capable of covalently binding to an amino group-containing compound, such as a protein were made to act on the electrode 11 provided with the intermediate layer 18. Actually, as the molecules 20, SUNBRIGHT OE-040CS, trade name, manufactured by NOF Corporation was used. This compound was dissolved in a dimethyl sulfoxide (DMSO) solution, and then the resulting solution was added in an amount of 100 µL dropwise onto the electrode 11 provided with albumin as the intermediate layer 18, and left to stand for one hour. Subsequently, the electrode 11 was washed with a phosphate buffer, and then used as an electrode for immobilization.

Next, a polyion complex technique, which is a technique for forming a conventional electrode film, was applied to the porous carbon electrode, the surface of which was hydrophilized by the UV ozonation treatment, and the electrode for immobilization.
As a control, an enzyme-immobilized electrode (Comparative Example 1) was fabricated by activating a porous carbon electrode by an UV ozonation treatment apparatus to hydrophilize the surface, and then performing immobilization of an enzyme using polyion complexes formed using PLL and PAAcNa. Comparison was carried out electrochemically. In the measurement, using an imidazole buffer solution containing 400 mM of glucose, constant potential measurement at 0.1 V and cyclic voltammetry were performed. A satisfactory catalytic current value was obtained in each case. Results of constant potential measurement after one hour showed that the value was 2.65 mA/cm² in Example 4, while the value was 2.35 mA/cm² in Comparative Example 1. Consequently, in Example 4, a significant difference from Comparative Example 1 is recognized, and the excellent result is obtained.
According to the fourth embodiment, the same advantages as those in the first embodiment can be obtained.

Next, a fifth embodiment of the present invention will be described. In the fifth embodiment, as a negative electrode of a biofuel cell, an enzyme-immobilized electrode according to any one of the first to fourth embodiments is used.

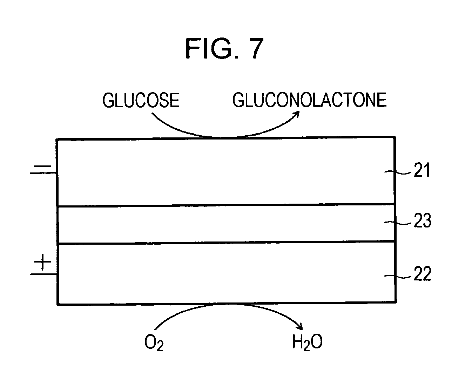

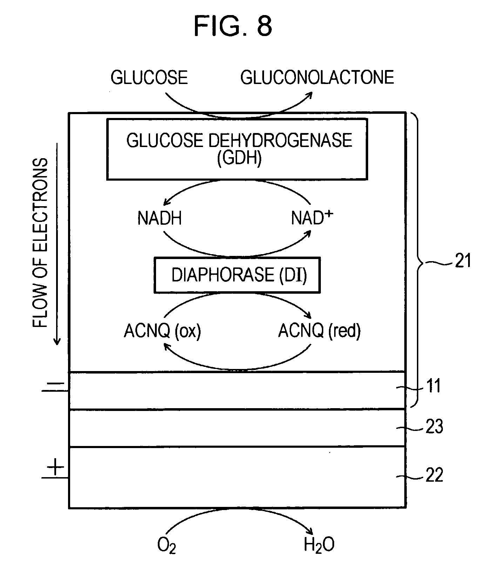

Fig. 7 schematically shows a biofuel cell according to the fifth embodiment. In the biofuel cell, glucose is used as the fuel. Fig. 8 schematically shows a detailed structure of a negative electrode of the biofuel cell, an example of a group of enzymes immobilized onto the negative electrode, and electron transfer reaction by the group of enzymes.
As shown in Fig. 7, the biofuel cell has a structure in which a negative electrode 21 and a positive electrode 22 are opposed to each other with an electrolyte layer 23 therebetween. The negative electrode 21 decomposes, by an enzyme, glucose supplied as the fuel to extract electrons and also generates protons (H⁺). The positive electrode 22 generates water by protons transported from the negative electrode 21 through the electrolyte layer 23, electrons transmitted from the negative electrode 21 through an external circuit, and oxygen, for example, in air.

The negative electrode 21 includes an enzyme involved in decomposition of glucose, a coenzyme (e.g., NAD⁺), the reduced form of which is generated in association with an oxidation reaction in the decomposition process of glucose, a coenzyme oxidase (e.g., diaphorase) which oxidizes the reduced form of the coenzyme (e.g., NADH), and an electron mediator (e.g., ACNQ) which receives electrons generated in association with oxidation of the coenzyme from the coenzyme oxidase and delivers the electrons to the electrode 11 are immobilized onto the electrode 11 (refer to Fig. 8), for example, composed of porous carbon or the like, in the same manner as that in the enzyme-immobilized electrode according to any one of the first to fourth embodiments.

As the enzyme involved in decomposition of glucose, for example, glucose dehydrogenase (GDH), preferably NAD-dependent glucose dehydrogenase, can be used. By the presence of the oxidase, for example, β-D-glucose can be oxidized to D-glucono-δ-lactone.
Furthermore, the D-glucono-δ-lactone can be decomposed into 2-keto-6-phospho-D-gluconate in the presence of two enzymes, gluconokinase and phosphogluconate dehydrogenase (PhGDH). That is, D-glucono-δ-lactone is hydrolyzed to D-gluconate, and D-gluconate is phosphorylated to 6-phospho-D-gluconate by hydrolysis of adenosine triphosphate (ATP) into adenosine diphosphate (ADP) and phosphoric acid in the presence of gluconokinase. The 6-phospho-D-gluconate is oxidized to 2-keto-6-phospho-D-gluconate by the action of the oxidase PhGDH.

Furthermore, besides the decomposition process described above, glucose can also be decomposed to CO₂ using sugar metabolism. The decomposition process using sugar metabolism is roughly classified into decomposition of glucose by the glycolytic pathway, generation of pyruvic acid, and the TCA cycle. These are widely known reaction systems.
An oxidation reaction in the decomposition process of a monosaccharide proceeds with a reduction reaction of a coenzyme. The coenzyme is almost determined by the enzyme on which the coenzyme acts, and in the case of GDH, NAD⁺ is used as the coenzyme. That is, when β-D-glucose is oxidized to D-glucono-δ-lactone by the action of GDH, NAD⁺ is reduced to NADH to generate H⁺.

The generated NADH is immediately oxidized to NAD⁺ in the presence of diaphorase (DI), and two electrons and H⁺ are generated. Consequently, two electrons and two H⁺s are generated per glucose molecule on one stage of oxidation reaction. Four electrons and four H⁺s in total are generated in two stages of oxidation reaction.
The electrons generated in the above-described process are transferred from diaphorase through the electron mediator to the electrode 11, and H⁺ is transported through the electrolyte layer 23 to the positive electrode 22.

The pH of each of the enzyme, the coenzyme, and the electronic mediator is preferably maintained at an optimum pH for the enzyme, for example, at about 7 by a buffer solution, such as a phosphate buffer or a Tris buffer, contained in the electrolyte layer 23 so that the electrode reaction is carried out efficiently and steadily. As the phosphate buffer, for example, NaH₂PO₄ or KH₂PO₄ is used. Furthermore, an excessively large or excessively small ionic strength (I.S.) adversely affects the enzyme activity. Taking also into consideration electrochemical responsiveness, an appropriate ionic strength, for example, of about 0.3, is preferable. However, each of the enzymes used has optimum values for pH and ionic strength, and the pH and the ionic strength are not limited to the values described above.

As an example, Fig. 8 shows the case where the enzyme involved in decomposition of glucose is glucose dehydrogenase (GDH), the coenzyme, the reduced form of which is generated in association with an oxidation reaction in the decomposition process of glucose is NAD⁺, the coenzyme oxidase which oxidizes NADH, i.e., the reduced form of the coenzyme, is diaphorase (DI), and the electron mediator which receives electrons generated in association with oxidation of the coenzyme from the coenzyme oxidase and delivers the electrons to the electrode 11 is ACNQ.

The positive electrode 22 is prepared by immobilizing an oxygen-decomposing enzyme, such as bilirubin oxidase, laccase, or ascorbate oxidase, onto a porous carbon electrode or the like. The outside portion (the portion opposite to the electrolyte layer 23) of the positive electrode 22 usually includes two gas diffusion layers composed of porous carbon. Preferably, in addition to the enzyme, an electron mediator is also immobilized onto the positive electrode 22 so that electrons are transferred between the positive electrode 22 and the electron mediator.
In the positive electrode 22, in the presence of the oxygen-decomposing enzyme, oxygen in air is reduced by H⁺ from the electrolyte layer 23 and electrons from the negative electrode 21 to generate water.

The electrolyte layer 23 is used to transport H⁺ generated at the negative electrode 21 to the positive electrode 22, and is composed of a material that does not have electron conductivity and that is capable of transporting H⁺. Specifically, the electrolyte layer 23 is, for example, composed of a perfluorocarbon sulfonate (PFS)-based resin film, a trifluorostyrene derivative copolymer film, a polybenzimidazole film impregnated with phosphoric acid, an aromatic polyether ketone sulfonic acid film, PSSA-PVA (polystyrene sulfonic acid-polyvinyl alcohol copolymer), PSSA-EVOH (polystyrene sulfonic acid-ethylene vinyl alcohol copolymer), or the like. Above all, an electrolyte layer 23 composed of an ion-exchange resin having fluorine-containing carbon sulfonic acid groups is preferable, and specifically, Nafion (trade name, DuPont, U.S.A.) is used.

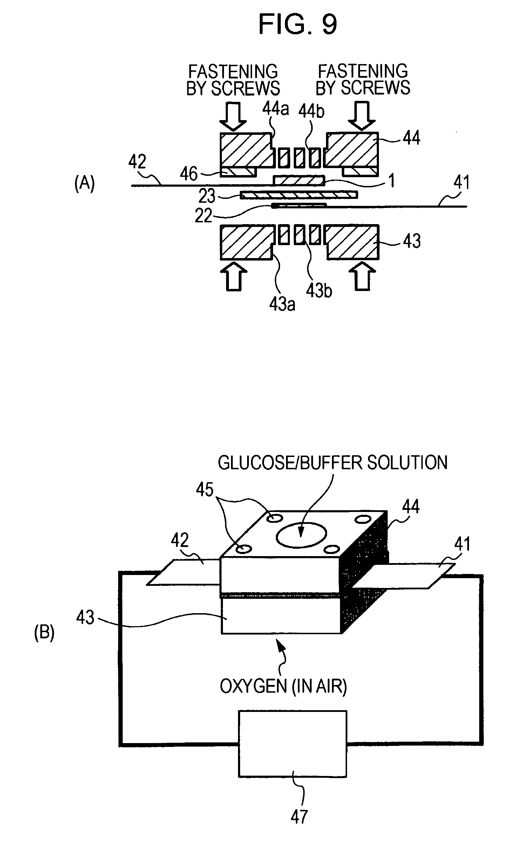

In the biofuel cell having the structure described above, when glucose is supplied to the negative electrode 21 side, the glucose is decomposed by the decomposing enzyme containing the oxidase. Since the oxidase is involved in the decomposition process of a monosaccharide, electrons and H⁺ can be generated at the negative electrode 21 side, thus generating a current between the negative electrode 21 and the positive electrode 22.
Next, specific structural example of a biofuel cell will be described.
As shown in Figs. 9(A) and (B), the biofuel cell has a structure in which a negative electrode 21 and a positive electrode 22 are opposed to each other with an electrolyte layer 23 therebetween. In this case, Ti current collectors 41 and 42 are respectively disposed under the positive electrode 22 and on the negative electrode 21, thus facilitating current collection. Reference numerals 43 and 44 each represent a fixing plate. The fixing plates 43 and 44 are fastened to each other by screws 45. The positive electrode 22, the negative electrode 21, the electrolyte layer 23, and the Ti current collectors 41 and 42 are entirely interposed between the fixing plates 43 and 44. A circular recess 43a for air intake is provided on one surface (outer surface) of the fixing plate 43, and many holes 43b passing through to the other surface are formed on the bottom of the recess 43a. The holes 43b serve as air-feed channels to the cathode electrode 22. Meanwhile, a circular recess 44a for fuel intake is provided on one surface (outer surface) of the fixing plate 44, and many holes 44b passing through to the other surface are formed on the bottom of the recess 44a. The holes 44b serve as fuel-feed channels to the negative electrode 21. A spacer 46 is provided at the periphery of the other surface of the fixing plate 44, and thereby, when the fixing plates 43 and 44 are fastened to each other by the screws 45, the fixing plates 43 and 44 can be disposed with a predetermined space therebetween.
As shown in Fig. 9(B), a load 47 is connected in between the Ti current collectors 41 and 42. For example, a glucose solution obtained by dissolving glucose in a phosphate buffer is placed as a fuel in the recess 44a of the fixing plate 44 to generate electric power.

According to the fifth embodiment, since the enzyme-immobilized electrode used as the negative electrode 21 is highly efficient, it is possible to realize a highly efficient biofuel cell. Furthermore, in order to increase the output of a biofuel cell, it is required to extract more electrons than 2 electrons from glucose as the fuel, and for that purpose, it is necessary to use an enzyme-immobilized electrode in which three or more types of enzymes are immobilized at appropriate positions. For example, by immobilizing three or more types of enzymes in the enzyme-immobilized electrode according to any of the first to fourth embodiments, such a requirement can also be satisfied.

Next, a biofuel cell according to a sixth embodiment of the present invention will be described.
In the biofuel cell, as the fuel, starch, which is a polysaccharide, is used. Furthermore, as starch is used as the fuel, glucoamylase, i.e. a decomposing enzyme which decomposes starch into glucose, is also immobilized onto the negative electrode 21.
In the biofuel cell, when starch is supplied as the fuel to the negative electrode 21 side, the starch is hydrolyzed by glucoamylase to glucose, and the glucose is further decomposed by glucose dehydrogenase. In association with the oxidation reaction in the decomposition process, NAD⁺ is reduced to generate NADH, and the NADH is oxidized by diaphorase and separated into two electrons, NAD⁺, and H⁺. Consequently, two electrons and two H⁺s are generated per glucose molecule on one stage of oxidation reaction. Four electrons and four H⁺s in total are generated in two stages of oxidation reaction. The electrons thus generated are transferred to the electrode 11 of the negative electrode 21, and H⁺ moves to the positive electrode 22 through the electrolyte layer 23. At the positive electrode 22, the H⁺ reacts with oxygen supplied from the outside and electrons transmitted from the negative electrode 21 through an external circuit to generate H₂O.
Except for what has been described above, the biofuel cell is the same as that of the biofuel cell according to the fifth embodiment.
According to the sixth embodiment, the same advantages as those in the fifth embodiment can be obtained. Another advantage is that, by using starch as the fuel, it is possible to increase the amount of electricity generated compared with the case where glucose is used as the fuel.

The embodiments of the present invention have been described above in detail. However, it is to be understood that the present invention is not limited to the embodiments described above, and various alterations are possible on the basis of the technical spirit of the present invention.
For example, the numerical values, structures, configurations, shapes, materials, etc. described in the embodiments are merely examples, and numerical values, structures, configurations, shapes, materials, etc. different from those described above may be used as necessary.
In addition, instead of phospholipid layers, it is also possible to use microcapsules, nanocapsules, artificial red blood cells, cells or crushed products of cells, organs, etc., emulsions, micelles, etc.

According to the present invention, it is possible to obtain a highly efficient enzyme-immobilized electrode in which one or a plurality of types of enzymes can be immobilized at optimal positions on the electrode. By using the enzyme-immobilized electrode, it is possible to realize a highly efficient fuel cell or an apparatus utilizing electrode reaction. Furthermore, by using such a highly efficient fuel cell, it is possible to realize a high-performance electronic device or the like.

## Claims

1. An enzyme-immobilized electrode **characterized by** comprising:
an electrode;
a phospholipid layer on the electrode; and
an enzyme immobilized onto the phospholipid layer.

2. The enzyme-immobilized electrode according to Claim 1, **characterized in that**, in addition to the enzyme, an electron mediator is immobilized onto the phospholipid layer.

3. The enzyme-immobilized electrode according to Claim 1, **characterized in that** the enzyme includes a plurality of types of enzymes.

4. The enzyme-immobilized electrode according to Claim 1, **characterized by** comprising an intermediate layer between the electrode and the phospholipid layer.

5. The enzyme-immobilized electrode according to Claim 4, **characterized in that** the intermediate layer comprises a biomacromolecule or a polyelectrolyte.

6. An enzyme-immobilized electrode **characterized by** comprising:
an electrode;
an intermediate layer on the electrode;
a phospholipid layer and/or a polyion complex on the intermediate layer; and
an enzyme immobilized onto the phospholipid layer and/or the polyion complex.

7. A fuel cell having a structure in which a positive electrode and a negative electrode are opposed to each other with a proton conductor therebetween, an enzyme being immobilized onto the positive electrode and/or the negative electrode, the fuel cell being **characterized in that** the positive electrode and/or the negative electrode is an enzyme-immobilized electrode comprising:
an electrode;
a phospholipid layer on the electrode; and
an enzyme immobilized onto the phospholipid layer.

8. The fuel cell according to Claim 7, **characterized in that**, in addition to the enzyme, an electron mediator is immobilized onto the phospholipid layer.

9. The fuel cell according to Claim 7, **characterized in that** the enzyme contains an oxidase which promotes oxidation of a monosaccharide and decomposes it.

10. The fuel cell according to Claim 7, **characterized in that** the enzyme contains a coenzyme oxidase which returns a coenzyme reduced in association with oxidation of the monosaccharide to an oxidized form and which transfers electrons to the negative electrode through an electron mediator.

11. The fuel cell according to Claim 10, **characterized in that** the oxidized form of the coenzyme is NAD⁺ and the coenzyme oxidase is diaphorase.

12. The fuel cell according to Claim 9, **characterized in that** the oxidase is NAD⁺-dependent glucose dehydrogenase.

13. The fuel cell according to Claim 7, **characterized in that** an enzyme is immobilized onto the negative electrode, and the enzyme contains a decomposing enzyme which promotes decomposition of a polysaccharide to produce a monosaccharide and an oxidase which promotes oxidation of the produced monosaccharide and decomposes it.

14. The fuel cell according to Claim 13, **characterized in that** the decomposing enzyme is glucoamylase, and the oxidase is NAD⁺-dependent glucose dehydrogenase.

15. A fuel cell having a structure in which a positive electrode and a negative electrode are opposed to each other with a proton conductor therebetween, an enzyme being immobilized onto the positive electrode and/or the negative electrode, the fuel cell being **characterized in that** the positive electrode and/or the negative electrode is an enzyme-immobilized electrode comprising:
an electrode;
an intermediate layer on the electrode;
a phospholipid layer and/or a polyion complex on the intermediate layer; and
an enzyme immobilized onto the phospholipid layer and/or the polyion complex.

16. An electronic device using one or a plurality of fuel cells, **characterized in that**:
at least one of the fuel cells has a structure in which a positive electrode and a negative electrode are opposed to each other with a proton conductor therebetween, an enzyme being immobilized onto the positive electrode and/or the negative electrode, and
the positive electrode and/or the negative electrode is an enzyme-immobilized electrode comprising:
an electrode;
a phospholipid layer on the electrode; and
an enzyme immobilized onto the phospholipid layer.

17. An electronic device using one or a plurality of fuel cells, **characterized in that**:
at least one of the fuel cells has a structure in which a positive electrode and a negative electrode are opposed to each other with a proton conductor therebetween, an enzyme being immobilized onto the positive electrode and/or the negative electrode, and
the positive electrode and/or the negative electrode is an enzyme-immobilized electrode comprising:
an electrode;
an intermediate layer on the electrode;
a phospholipid layer and/or a polyion complex on the intermediate layer; and
an enzyme immobilized onto the phospholipid layer and/or the polyion complex.

18. An apparatus utilizing enzyme reaction comprising an enzyme-immobilized electrode, the apparatus utilizing enzyme reaction being **characterized in that** the enzyme-immobilized electrode comprises:
an electrode;
a phospholipid layer on the electrode; and
an enzyme immobilized onto the phospholipid layer.

19. An apparatus utilizing enzyme reaction comprising an enzyme-immobilized electrode, the apparatus utilizing enzyme reaction being **characterized in that** the enzyme-immobilized electrode comprises:
an electrode;
an intermediate layer on the electrode;
a phospholipid layer and/or a polyion complex on the intermediate layer; and
an enzyme immobilized onto the phospholipid layer and/or the polyion complex.

20. An enzyme-immobilized substrate **characterized by** comprising:
a substrate;
a phospholipid layer on the substrate; and
an enzyme immobilized onto the phospholipid layer.

21. An enzyme-immobilized substrate **characterized by** comprising:
a substrate;
an intermediate layer on the substrate;
a phospholipid layer and/or a polyion complex on the intermediate layer; and
an enzyme immobilized onto the phospholipid layer and/or the polyion complex.
